# EUROPEAN PATENT APPLICATION

(11) **EP 4 520 263 A2**
(43) Date of publication of application: **12.03.2025**
(21) Application number: 24194719.1
(22) Date of filing: 15.08.2024
(51) Int. Cl.: A61B 5/06, A61B 5/287, A61B 5/0538, A61B 5/00

(54) **MAGNETIC SENSOR SENSITIVITY ENHANCEMENT**

(30) Priority: 16.08.2023 US 202363519970 P; 16.07.2024 US 202418774675
(71) Applicant: Biosense Webster (Israel) Ltd., 2066717 Yokneam (IL)
(72) Inventor: RODRIGUEZ SOTO, Juan, Irvine, 92618 (US); VAN NIEKERK, Pieter Emmelius, Irvine, 92618 (US); EBRAHIMI, Babak, Irvine, 92618 (US); ABBAS, Mohammad, Irvine, 92618 (US)
(74) Representative: Carpmaels & Ransford LLP

(57) **Abstract**

The disclosed technology includes a magnetic location sensor which may comprise first and second spiral coils that can be disposed on a planar substrate. The first and second spiral coils may be disposed about respective first and second center axes, wherein each of the first and second coils can be configured to generate a voltage when subjected to a magnetic field. The magnetic location sensor may also comprise a high-magnetic-permeability planar member that can be disposed near one of the first and second spiral coils. The high-magnetic-permeability planar member can be disposed on the magnetic location sensor such that if the planar substrate is wrapped around a cylindrical substrate, the high-magnetic-permeability planar member may be disposed on an intersection of the first and second center axes.

## Description

### CROSS-REFERENCE TO RELATED APPLICATION

This application claims, under 35 U.S.C. § 119(e), priority to and the benefit of U.S. Provisional Patent Application No. 63/519,970, filed August 16, 2023 (Attorney Docket No.: BIO6837USPSP1 - 253757.000385), the entire contents of which are incorporated herein by reference.

### FIELD

The present invention relates generally to magnetic sensors and more specifically a magnetically permeable material disposed near a magnetic sensor which can help to increase the magnetic sensitivity of the magnetic sensor.

### BACKGROUND

Magnetic sensors are sensors that are capable of detecting a magnitude and variations of a radiated magnetic field and outputting an electrical signal indicative of the detected magnitude and variations of the radiated magnetic field. Magnetic sensors include, but are not limited to, coiled magnetic sensors, Reed switches, Hall effect elements, magnetoresistance elements, superconducting quantum interference devices, and the like. Medical positioning systems leverage magnetic sensors to track a medical device within the body of a patient. Conventionally, to locate the medical device within the body of a patient, medical positioning systems include magnetic field generators configured to radiate one or more magnetic fields to induce an electrical signal from the magnetic sensor. The accuracy and dependability of the magnetic sensor is directly proportional to the strength of the induced voltage emitted as an electrical signal by the magnetic sensor. Resultantly, a greater induced voltage from the magnetic sensor provides greater resolution and accuracy via a stronger electrical signal to the medical positioning system.

One way to increase the strength of the induced voltage emitted by the magnetic sensor is to increase the number of windings or loops on the magnetic sensor to increase the sensor's sensitivity. A problem with this methodology, however, is that additional windings or loops generally increases the size of the magnetic sensor. This makes some magnetic sensors oversized and inapplicable to areas of a medical device wherein position sensing may be desired. For example, the end effector of a catheter may not be able to support current magnetic sensors, which is attributed to the large dimensional footprint of the magnetic sensor resulting from increasing the numbers of windings or loops.

As will be appreciated, magnetic sensors with large dimensional footprints or lower induced voltages can result in incompatibility with medical devices and less accurate position data relayed to medical positioning systems. The technology disclosed herein addresses the aforementioned challenges.

### SUMMARY

There is provided, in accordance with an example of the present technology, a magnetic location sensor which may include first and second spiral coils that can be disposed on a planar substrate. The first and second spiral coils may be disposed about respective first and second center axes, wherein each of the first and second coils can be configured to generate a voltage when subjected to a magnetic field. The magnetic location sensor may also include a high-magnetic-permeability planar member that can be disposed near one of the first and second spiral coils. The high-magnetic-permeability planar member can be disposed on the magnetic location sensor such that if the planar substrate is wrapped around a cylindrical substrate, the high-magnetic-permeability planar member may be disposed on an intersection of the first and second center axes.

There is provided, in accordance with an example of the present technology, an electrode assembly which may include a planar substrate, an electrode, an electromagnetic coil, and a member. The planar substrate may extend along a plane. The electrode may be disposed on the planar substrate and can be configured to detect electrophysiological signals. The electromagnetic coil can be disposed on the planar substrate and can be configured to generate a voltage when subject to a magnetic field. The member may comprise a high-magnetic-permeability material that can be disposed near the electromagnetic coil.

There is provided, in accordance with an example of the present technology, an end effector that may include a plurality of electrodes, a coil, and a member. The plurality of electrodes may be disposed along the end effector and can be configured to detect electrophysiological signals. The coil may be disposed along the end effector and can be configured to generate a voltage when subjected to a magnetic field. The member can be disposed near the coil and may include a high-magnetic-permeability material.

The end effector may have a generally planar shape wherein the plurality of electrodes may be disposed along the generally planar shape in an arrangement of oner or more rows. The coil may be disposed along an outer periphery of the general planar shape of the end effector.

There is provided, in accordance with an example of the present technology, an end effector that may include a body, a plurality of first electrode assemblies, and a plurality of second electrode assemblies. The body may have a generally planar shape. The plurality of first electrode assemblies may be disposed along the body and can be configured to detect electrophysiological signals. The second plurality of electrode assemblies may be disposed along the body of the end effector.

Each electrode assembly of the plurality of second electrode assemblies may include an electrode, a coil, and a member. The electrode may be configured to detect electrophysiological signals. The coil may be disposed on the body of the end effector and can be configured to generate a voltage when subject to a magnetic field. The coil may include a spiral shape that can have a width approximately equal to a width of the electrode. The member may include a high-magnetic-permeability material.

There is provided, in accordance with an example of the present technology, an end effector that may include a plurality of flexible spines and a plurality of electrode assemblies. The plurality of flexible spines can be configured to transition the end effector between an expanded configuration and a collapsed configuration. The plurality of electrode assemblies may be disposed along the plurality of flexible spines and each of the plurality of electrode assemblies can include a substrate, an electrode, a coil, and a member.

The substrate may extend at least partially along a spine of the plurality of flexible spines. The electrode may be disposed on a first side of the substrate and can be configured to detect electrophysiological signals. The coil may be disposed on a second side of the substrate and can be configured to generate a voltage when subject to a magnetic field. The member may include a high-magnetic-permeability material.

Additional features, functionalities, and applications of the disclosed technology are discussed in more detail herein.

### BRIEF DESCRIPTION OF THE DRAWINGS

FIG. 1 is a schematic pictorial illustration of a medical system including a catheter and handle, in accordance with an example of the disclosed technology;
FIG. 2 is a schematic pictorial illustration of a catheter and handle, in accordance with an example of the disclosed technology;
FIG. 3 is a detail view of an end effector of the catheter, in accordance with an example of the disclosed technology;
FIG. 4A is a detail view of a magnetic sensor, in accordance with an example of the disclosed technology;
FIG. 4B is a detail view of a magnetic sensor with a high-magnetic-permeability material affixed to the magnetic sensor in a first configuration, in accordance with an example of the disclosed technology
FIG. 4C is an alternate detail view of a magnetic sensor with a high-magnetic-permeability material affixed to the magnetic sensor in a second configuration, in accordance with an example of the disclosed technology;
FIG. 4D is a perspective view of a high-magnetic-permeability material disposed between two magnetic sensors, in accordance with an example of the disclosed technology;
FIG. 4E is perspective view of a magnetic sensor and a high-magnetic-permeability material affixed to the magnetic sensor, in accordance with an example of the disclosed technology;
FIG. 5A is a photograph of a magnetic sensor assembly with a high-magnetic-permeability material disposed between two magnetic sensors, in accordance with an example of the disclosed technology;
FIG. 5B is an X-ray view of the magnetic sensor assembly of FIG. 5A, in accordance with an example of the disclosed technology;
FIG. 5C is a photograph of a magnetic sensor assembly with a high-magnetic-permeability material disposed between two magnetic sensors, in accordance with another example of the disclosed technology;
FIG. 5D is an X-ray view of the magnetic sensor assembly of FIG. 5C, in accordance with an example of the disclosed technology;
FIG. 6 is a multivariable graph showing the relationship between concentrations of a high-magnetic-permeability material with various cores, in accordance with examples of the disclosed technology;
FIG. 7 is a multi-variable graph showing the relationship between magnetic sensitivity and concentrations of high-magnetic-permeability materials, in accordance with examples of the disclosed technology;
FIG. 8 is a table showing magnetic sensitivity of various concentrations of high-magnetic-permeability materials for three sample electrode assemblies at predetermined frequencies, in accordance with an example of the disclosed technology;
FIG. 9A is a perspective view of an exemplary end effector assembly including electrodes and a magnetic sensor assembly, in accordance with an example of the disclosed technology;
FIG. 9B is a perspective view of the magnetic sensor assembly of FIG. 9A, in accordance with an example of the disclosed technology;
FIGs. 9C and 9D are perspective views of the magnetic sensor of FIGs. 9A and 9B and high-magnetic-permeability materials, in accordance with an example of the disclosed technology;
FIG. 10 is a perspective view of another exemplary end effector assembly with high-magnetic-permeability materials in an alternate configuration, in accordance with an example of the disclosed technology;
FIG. 11A is a top view of an electrode assembly having a high-magnetic-permeability material, in accordance with an example of the disclosed technology;
FIG. 11B, is a cross-sectional view of an electrode assembly having a high-magnetic-permeability material shown in FIG. 11A, in accordance with an example of the disclosed technology;
FIG. 11C, is a bottom view of an electrode assembly having a high-magnetic-permeability material, in accordance with an example of the disclosed technology;
FIG. 12A is a section view of an electrode assembly having a high-magnetic-permeability material, in accordance with an example of the disclosed technology;
FIG. 12B is a cross-sectional view of an electrode assembly having a high-magnetic-permeability material, in accordance with an example of the disclosed technology; and
FIG. 12C is another section view of an electrode assembly having a high-magnetic-permeability material, in accordance with an example of the disclosed technology.

### DETAILED DESCRIPTION

The following detailed description should be read with reference to the drawings, in which like elements in different drawings are identically numbered. The drawings, which are not necessarily to scale, depict selected examples and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "physician" can include a doctor, surgeon, technician, scientist, operator, or any other individual or delivery instrumentation associated with delivery of a multi-electrode catheter for the treatment of drug refractory atrial fibrillation to a subject.

It is noted that the electrodes described and illustrated herein are not limited to mapping (i.e., sensing signals or recording signals) but can be used to deliver energy such as RF (alternating cycle) or IRE (DC pulses) in bipolar or unipolar mode alone or in combination with the mapping or sensing function. In the application for IRE, the electrodes can be configured to deliver at least 900V per electrode with a current of at least 20 amperes over a number of pulses sufficient to cause cell apoptosis

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as reversable or irreversible electroporation (IRE), referred throughout this disclosure interchangeably as pulsed electric field (PEF) and pulsed field ablation (PFA), or thermal energy such as radiofrequency (RF) ablation or cryoablation. Ablating or ablation as it relates to the devices and corresponding systems of this disclosure is used throughout this disclosure in reference to thermal or non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

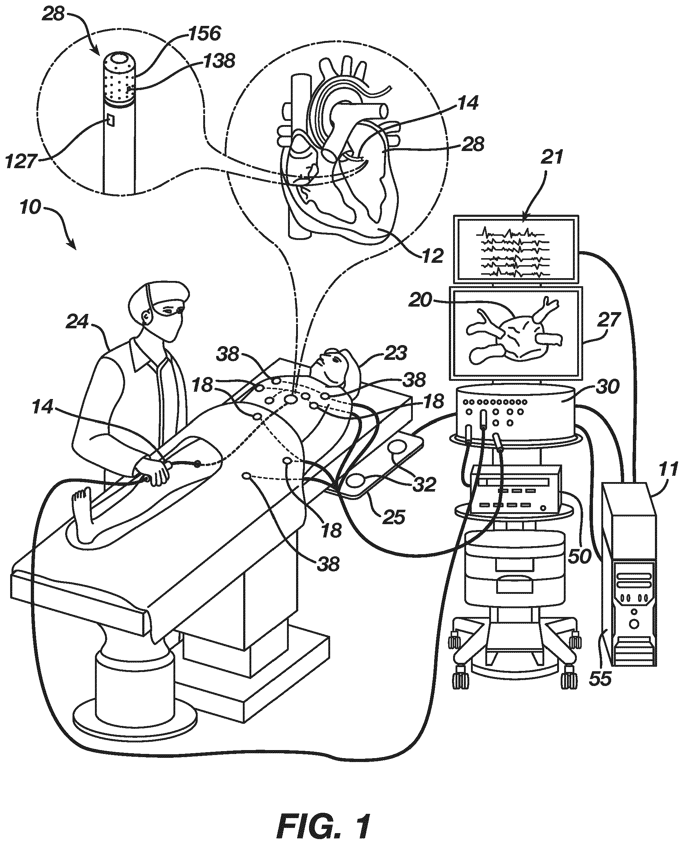

FIG. 1 shows an example system (10) that can include a catheter (14). The catheter (14) can be configured for navigating to a patient's (23) heart (12) and for mapping and/or ablation of myocardial tissue. System (10) can be configured for multiple catheters, which are percutaneously inserted by physician (24) through the patient's (23) vascular system into a chamber or vascular structure of a heart (12). Typically, a catheter (14) is inserted into the left or right atrium near a desired location in heart (12). Thereafter, a plurality of catheters (14) can be inserted into the catheter (14) so as to arrive at the desired location. The plurality of catheters can include catheters dedicated for sensing Intracardiac Electrogram (IEGM) signals, catheters dedicated for ablating and/or catheters dedicated for both sensing and ablating. An example catheter (14) that is configured for sensing IEGM is illustrated herein. Physician (24) brings a distal tip of a mapping catheter into contact with the heart wall for sensing a target site in heart (12). The end effector of the mapping catheter may include a basket catheter, a planar catheter, a focal catheter, a balloon catheter, etc. For ablation, physician (24) similarly brings a distal end of an ablation catheter to a target site for ablating. Similarly, the end effector of the ablation catheter may include a basket catheter, a planar catheter, a focal catheter, a balloon catheter, etc.

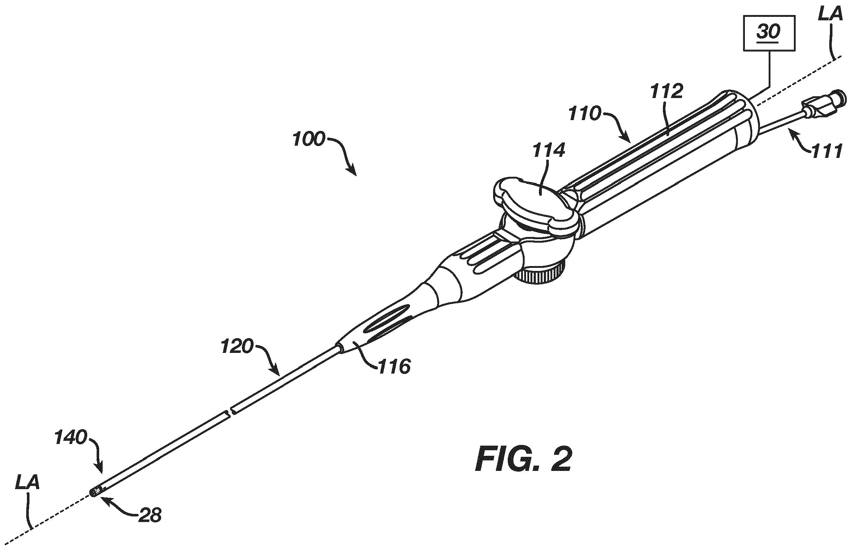

Catheter (14) is an exemplary catheter that includes one and preferably multiple electrodes (127) optionally distributed over a distal end (28) of the catheter (14) for tracking position and orientation of catheter (14). Optionally and preferably, electrodes (127) can be magnetic based position sensors including three magnetic coils for sensing three-dimensional (3D) position and orientation. The catheter (14) can further include a position sensor that can be configured for impedance-based position tracking. As shown in FIG. 2, catheter (14) can include a handle (100) at a proximal end thereof.

Magnetic based position sensor (e.g., electrodes 127) may be operated together with a location pad (25) including a plurality of magnetic coils (32) configured to generate magnetic fields in a predefined working volume. Real time position of distal end (28) of catheter (14) may be tracked based on magnetic fields generated with location pad (25) and sensed by magnetic-based position sensors (e.g., electrodes 127). Details of the magnetic based position sensing technology are described in U.S. Patent Nos. 5,391,199; 5,443,489; 5,558,091; 6,172,499; 6,239,724; 6,332,089; 6,484,118; 6,618,612; 6,690,963; 6,788,967; 6,892,091, each of which are incorporated herein by reference.

System (10) can include one or more electrode patches (38) positioned for skin contact on patient (23) to establish location reference for location pad (25) as well as impedance-based tracking of electrodes (not shown). For impedance-based tracking, electrical current is directed toward electrodes and sensed at electrode skin patches (38) so that the location of each electrode can be triangulated via the electrode patches (38). Details of the impedance-based location tracking technology are described in US Patent Nos. 7,536,218; 7,756,576; 7,848,787; 7,869,865; and 8,456,182, each of which are incorporated herein by reference.

A recorder (11) displays electrograms (21) captured with body surface ECG electrodes (18) and intracardiac electrograms (IEGM) captured with electrodes (138) of the catheter (14). Recorder (11) may include pacing capability for pacing the heart rhythm and/or may be electrically connected to a standalone pacer.

System (10) may include an ablation energy generator (50) that is adapted to conduct ablative energy to one or more of electrodes at a distal tip of a catheter configured for ablating. Energy produced by ablation energy generator (50) may include, but is not limited to, radiofrequency (RF) energy or pulsed-field ablation (PFA) energy, including monopolar or bipolar high-voltage DC pulses as may be used to effect irreversible electroporation (IRE), or combinations thereof.

Patient interface unit (PIU) (30) is an interface configured to establish electrical communication between catheters, electrophysiological equipment, power supply and a workstation (55) for controlling operation of system (10). Electrophysiological equipment of system (10) may include for example, multiple catheters, location pad (25), body surface ECG electrodes (18), electrode patches (38), ablation energy generator (50), and recorder (11). Optionally and preferably, PIU (30) additionally includes processing capability for implementing real-time computations of location of the catheters and for performing ECG calculations.

Workstation (55) includes memory, processor unit with memory or storage with appropriate operating software loaded therein, and user interface capability. Workstation (55) may provide multiple functions, optionally including (1) modeling the endocardial anatomy in three-dimensions (3D) and rendering the model or anatomical map (20) for display on a display device (27), (2) displaying on display device (27) activation sequences (or other data) compiled from recorded electrograms (21) in representative visual indicia or imagery superimposed on the rendered anatomical map (20), (3) displaying real-time location and orientation of multiple catheters within the heart chamber, and (4) displaying on display device (27) sites of interest such as places where ablation energy has been applied. One commercial product embodying elements of the system (10) is available as the CARTO^{™} 3 System, available from Biosense Webster, Inc., 31 Technology Drive, Suite 200, Irvine, CA 92618, USA.

FIG. 2 is a schematic pictorial illustration showing a perspective view of a catheter (100) according to the disclosed technology. The catheter (100) can be or include the same catheter (14) previously described. The catheter (100) can have a handle (110) at a proximal end of the catheter (100) and an end effector 140 disposed on a distal end (28) of the catheter (100). The catheter (100) can be configured for insertion and navigation of a patient's vasculature to position the distal end (28) of the catheter (100) in a chamber of the patient's heart (12). The catheter assembly (100) can include a handle (110), a tubular shaft (120) extending distally from handle (110), an end effector (140) located at a distal end (28) of the tubular shaft (120), and a deflection drive actuator (114) associated with handle (110). As will be described in greater detail below, end effector (140) includes various components configured to deliver ablative energy to targeted tissue sites, provide electrophysiology (EP) mapping functionality, detect external forces imparted on end effector (140), detect the location of the end effector (140), and/or disperse irrigation fluid. The deflection drive actuator (114) is rotatable relative to a casing (112) of the handle (110) and can cause the end effector (140) and a distal portion of tubular shaft (120) to deflect away from a central longitudinal axis (LA) extending along the tubular shaft (120). Various suitable components may be coupled with the deflection drive actuator (114) and tubular shaft (120) to provide such functionality that will be apparent to those skilled in the art in view of the teachings herein.

As mentioned previously, at least the distal end (28) of the sheath (106) can include one or more electrodes (127) configured for position sensing (e.g., magnetic- or impedance-based position tracking). The distal end of the catheter (100) can be configured to deflect in one or more directions to enable steering of the catheter (100). For example, the catheter (100) can include one or more pull wires (not shown) that are attached to the deflection drive actuator (114) configured to pull the one or more pull wires when actuated to cause the distal end (28) to deflect as will be appreciated by those skilled in the art.

The handle (100) can further include an irrigation supply tube (111) that can direct saline or other irrigation fluid from an irrigation supply and into the handle (110) for delivery to the distal end (28). The handle (100) can also include an electrical connector (not pictured) that can be electrically connected to the one or more electrodes (127) and can be configured for connection to the PIU (30) via cable or other suitable electrical connection shown in FIG. 2.

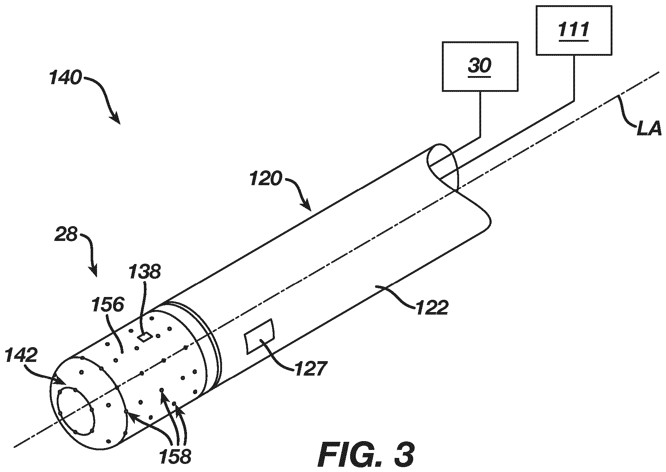

FIG. 3 illustrates an example end effector (140) of a catheter (100). The end effector (140) illustrated in FIG. 3, however, is offered for illustrative purposes and should not be construed as limiting. It will be appreciated that various other types of end effectors can be used in placed of the end effector (140) illustrated in FIG. 3 such as basket catheters, planar catheters, balloon catheters, circular catheters, catheters having multiple spines, and the like. As shown in FIG. 3, the distal tip (28) of the handle (100) may include a cylindraceous body (156) with a dome tip. The cylindraceous body (156) and the dome tip may be formed of an electrically conductive material, such as metal and form an electrode configured to deliver ablative energy to tissue. A plurality of openings (158) can be formed through the cylindraceous body (156) that are in communication with the hollow interior of distal tip (28). Openings (158) may allow irrigation fluid to be communicated from the interior of distal tip (28) out through the cylindraceous body (156) via the irrigation supply tube (111), as shown in FIG. 2 and FIG. 3. The cylindraceous body (156) and the dome tip may also be operable to apply ablative electrical energy to tissue to thereby ablate the tissue. Such ablative electrical energy may be communicated from the ablation energy generator (50). The distal tip (28) may also include one or more thermocouples (not shown) that can be configured to provide temperature sensing capabilities. This may prevent overheating of distal tip member (142) or adjacent tissue.

As shown in FIG. 3, the distal tip member (28) of the catheter (100) can also include one or more electrophysiological (EP) mapping microelectrodes (138) mounted to the cylindraceous body (156). EP mapping microelectrodes (138) can be configured to pick up electrical potentials from tissue that comes into contact with EP mapping microelectrodes (138). EP mapping microelectrodes (138) may thus be used to determine locations of aberrant electrical activity in tissue within a cardiovascular anatomical structure (e.g., pulmonary vein, etc.). Signals picked up by EP mapping microelectrodes (138) may be communicated via cable to the PIU (30). The PIU (30) may process the EP mapping signals and provide the physician (PH) with corresponding feedback indicating the locations of aberrant electrical activity in accordance with the teachings of various references cited herein.

When the cylindraceous body (156) is formed of an electrically conductive material to provide ablative electrical energy for tissue ablation, an electrically insulating material may be interposed between cylindraceous body (156) and EP mapping microelectrodes (138) to thereby electrically isolate EP mapping microelectrodes (138) from cylindraceous body (156). EP mapping microelectrodes (138) may be constructed and operable in accordance with the teachings of various patent references cited herein. While only one EP mapping microelectrode (138) is shown, distal tip (28) may include two or more EP mapping microelectrodes (138). Alternatively, distal tip member (142) may lack EP mapping microelectrodes (138) altogether.

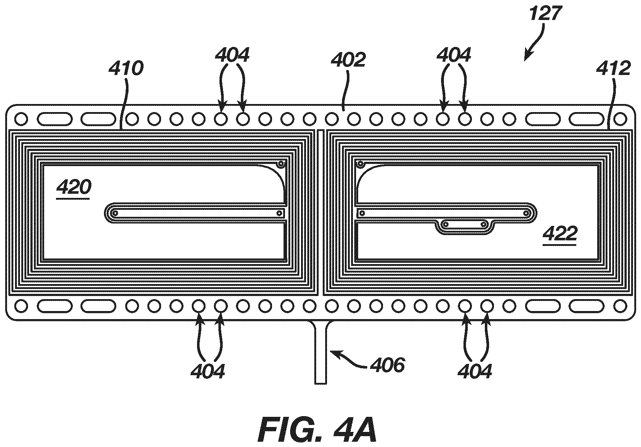

FIG. 4A is a detail view of a position sensor (127) (sometimes referred to herein as a "magnetic position sensor" or "magnetic sensor"). As shown in FIG 4A, the position sensor (127) can include a flexible substrate (402) having a plurality of openings (404) formed therethrough. The flexible substrate (402) may be formed of polyimide material, liquid crystal polymer (LCP) material, and/or any other suitable kind(s) of material(s). The flexible substrate (402) may also include a tail portion (406). Electrode pads (420, 422) may be configured to contact blood and measure impedance of the blood, which can be used for sensing by impedance or for measurement of electrophysiological mapping reference signals in blood. In some embodiments, the electrode pads (420, 422) may be used to obtain impedance measurements that can be used to provide location sensing as disclosed in US Patent Nos. 7,869,865; and 8,456,182, the disclosures of each of which is incorporated by reference herein, in its entirety. As one skilled in the art will appreciate, the electrode pads (420, 422) can behave as a second type of navigation sensor. In certain scenarios, using the electrode pads (420,422) as a second type of navigation sensor might not be as accurate as the first type of navigation sensor (e.g., electromagnetic type location sensor) and therefore may require a location matrix to be generated before this second type of navigation sensor (e.g., impedance location sensing) can be viewed by the operator.

Resultantly, impedance signals from the electrode pads (420, 422) may be used in conjunction with signals from coil-based sensors (e.g., a navigation sensor formed by a first coil and a second coil (410, 412) to create a location matrix; similar to the teachings provided above. In some embodiments, electrode pads (420, 422) are used to contact tissue and thereby measure the impedance of the tissue for other purposes. While the position sensor (127) includes only two electrode pads (420, 422) in this example, the position sensor (127) may instead include just one electrode pad or more than two electrode pads.

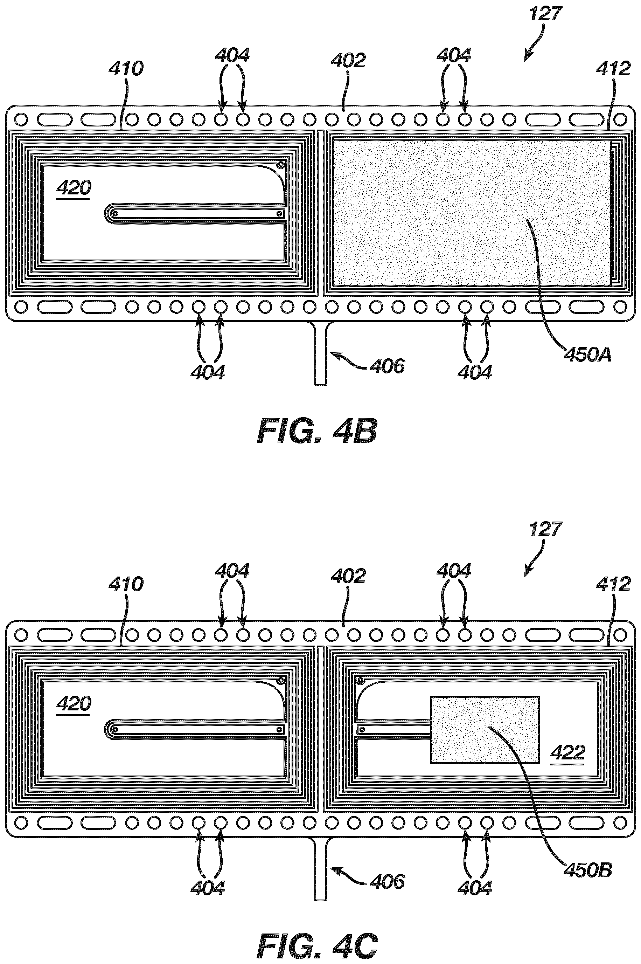

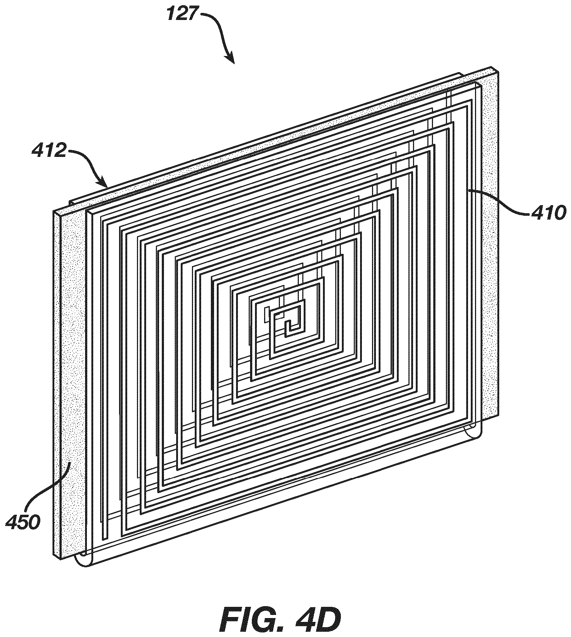

FIG. 4B, FIG. 4C, and FIG. 4D show detail views of the position sensor (127) with a high-magnetic-permeability material (450A, 450B) affixed to the position sensor (127) in one or more configurations. As one skilled in the art will appreciate, high-magnetic-permeability materials can easily align themselves with an applied magnetic field. "Magnetic permeability" as used herein, unless indicated to the contrary, refers to the ability of a material or element to support the formation of a magnetic field within itself. Furthermore, the term "magnetic permeability" can also be understood as the degree of magnetization that a material obtains in response to an applied magnetic field. A material with a "high permeability" or "high magnetic permeability" as used herein, unless indicated to the contrary, means any material having a relative magnetic permeability above the relative magnetic permeability of Martensitic stainless steel.

The high-magnetic permeability material described herein, with respect to the present technology, can be made of any material, with materials of higher magnetic permeability being more suitable. Magnetic field lines preferentially travel through materials with high permeability. In various embodiments of the present technology, µ (Mu) -metals, amorphous metal alloys (also known as metallic glass alloys), nanocrystalline metals or 99.95% pure iron may be used. One particular branch of Mu metals and Metglas^{®} amorphous alloys (METGLAS is a registered trademark of Metglas, Inc. of Conway, South Carolina) are both particularly well suited for use with cores of the present disclosure. As compared to air with a magnetic permeability equal to one (i.e., µ = 1), it has been found that Mu metals have a relative magnetic permeability of approximately 50,000 while 99.95% pure iron has a relative magnetic permeability of approximately 200,000.

As shown in FIG. 4B, the high-magnetic-permeability planar member (450A) may be attached or placed over the electrode pad (422) and at least one of the first coil (410) or the second coil (412) of the position sensor (127) (FIG. 4B shows the high-magnetic-permeability planar member (450A) placed over the second coil (412)). The high-magnetic-permeability planar member (450A) can have a dimensional footprint that can be greater than, less than, or equal to an area of at least one of the first and second spiral coils (410, 412). It should be appreciated that the high-magnetic-permeability planar member (450A) may also be placed or attached over the opposite electrode pad (420) and the first coil (410) with the same dimensional footprint as shown in FIG. 4B. While the position sensor (127) is shown in a flat pattern, it must be noted that the sensor (127) can be wrapped around a cylindrical member in its final operational form. As mentioned previously, affixing or placing the high-magnetic-permeability planar member (450A) against the first and/or second coil (410,412) of the position sensor (127) can vastly increase the magnetic sensitivity of the position sensor (127), specifically due to the high magnetic permeability properties of the material composition of the high-magnetic-permeability planar member (450A). In some embodiments, the high-magnetic-permeability planar member (450A) can include Mu metals and/or Metglas^{®} amorphous alloys (METGLAS is a registered trademark of Metglas, Inc. of Conway, South Carolina) as mentioned previously. Increasing the magnetic sensitivity of the position sensor (127) can be advantageous as it can provide a physician (24), for example, greater positional accuracy of the distal end (28) of the handle (100) when inside the body of the patient (23), such as the patient's (23) vasculature. Furthermore, by increasing the magnetic sensitivity of the position sensor (127), smaller position sensors (127) can be used to determine the position of the end effector (140).

As shown in FIG 4C, the high-magnetic-permeability planar member (450B), in some examples, may be attached or placed over the electrode pad (422) and the second coil (412) of the position sensor (127), with a dimensional footprint that can be less than or equal to an area of at least one of the first and second spiral coils (410, 412). In other words, FIG. 4C illustrates a high-magnetic-permeability planar member (450B) having an area that is less than the area of the high-magnetic-permeability planar member (450A) shown in FIG. 4B. As one skilled in the art will appreciate, adjustments to the size of the high-magnetic-permeability planar member (450B) may impact the overall magnetic sensitivity of the position sensor (127). In other words, the size of the high-magnetic-permeability planar member (450B) will impact the overall magnetic sensitivity observed in the position sensor (127), which will be described in greater detail herein.

FIG. 4D is a perspective view of a high magnetic permeability material disposed between two position sensors (127). In some examples, the high-magnetic-permeability material (450) may be compressed between the first and second coils (410, 412), as shown in FIG. 4D. As one skilled in the art will appreciate, by placing the high-magnetic-permeability material (450) between the first and second coils (410, 412), the magnetic sensitivity of both the first and second coils (410, 412) can be increased. Furthermore, optimization of the size of the first and second coils (410,412) in combination with affixing or placing a high-magnetic-permeability material (450) within or onto the position sensor (127) can increase magnetic sensitivity of the position sensor (127), as discussed previously.

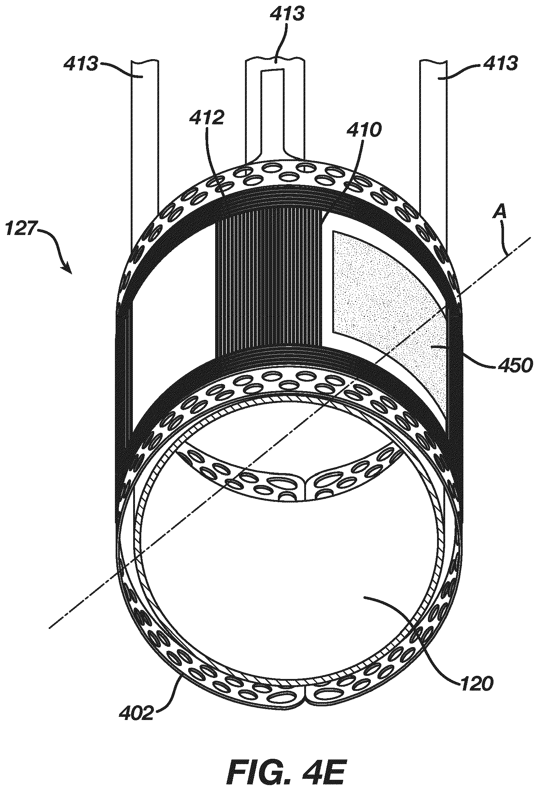

FIG. 4E is perspective view of a position sensor (127) and a high magnetic permeability material affixed to the position sensor (127). As mentioned previously, the position sensor (127) can include a flexible substrate (402), which can be deformed to conform the position sensor (127) to a plethora of shapes, configurations, and the like. In some embodiments, as shown in FIG. 4E, the flexible substrate (402) and high-magnetic-permeability material (450) can be deformed around the tubular shaft (120). The high-magnetic-permeability material (450) can be positioned on the position sensor (127) such that the high-magnetic-permeability material (450) is aligned along an axis (A) that passes through the center of the first and second coils (410, 412). In this way, the high-magnetic-permeability material (450) can be configured to increase the sensitivity of both the first and second coils (410, 412). The position sensor (127) can also include one or more wire leads (413) that can be configured to relay electrical signals obtained from the position sensor (127) to the system (10).

Although shown in FIGs. 4B-4E as having a first coil (410) and a second coil (412), the disclosed technology can be applicable to position sensors (127) having a single coil or a plurality of coils. For example, a position sensor (127) may have a single coil with a high magnetic permeability material disposed on or nearby the single coil. Alternatively, the position sensor (127) can have two, three, four, five, or even more coils with each, some, or only one of the coils having a high magnetic permeability material disposed on or nearby the coils. Thus, although specific examples are disclosed herein, it will be appreciated that several variations of position sensors (127) are also considered within the scope of this disclosure.

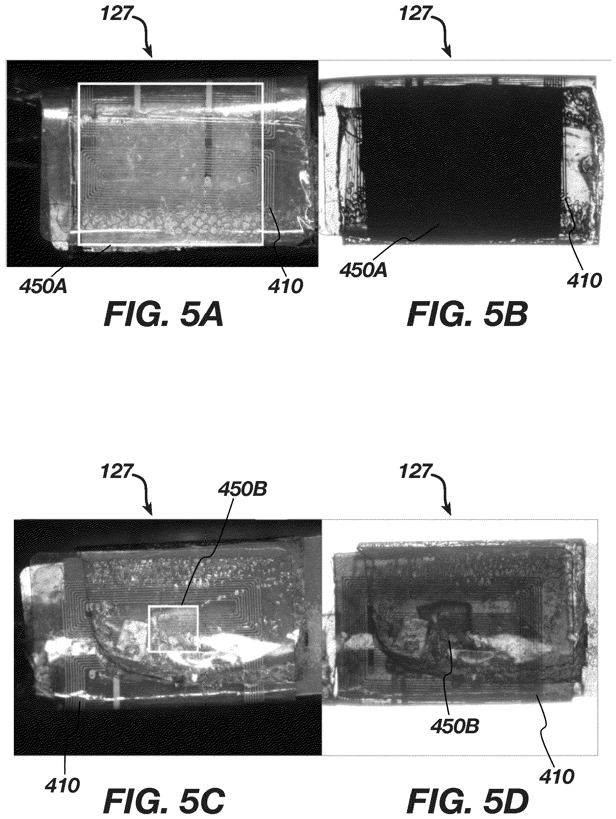

FIG. 5A is a photograph of a position sensor assembly with a high magnetic permeability material disposed between two position sensors (127). FIG. 5B is a radiograph of the position sensor assembly shown in FIG. 5A. Similar to FIG. 4B, the high-magnetic-permeability planar member (450A) as shown in FIG. 5A and FIG. 5B, has a dimensional footprint that can be greater than or equal to an area of the one of the first and second spiral coils (410, 412). FIG. 5C is a photograph of another exemplary position sensor assembly with a high magnetic permeability material disposed between two position sensors. FIG 5D is a radiograph of the position sensor assembly shown in FIG 5C. Similar to FIG. 4C, the high-magnetic-permeability material (450B) shown in FIGs. 5C and 5D has a dimensional footprint that can be less than an area of the one of the first and second spiral coils (410, 412) as shown in FIG. 5C and FIG. 5D.

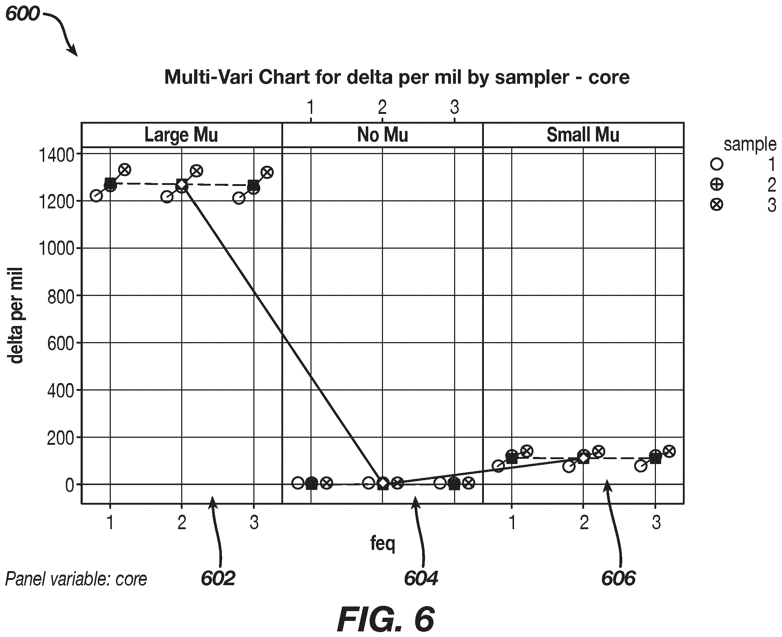

FIG. 6 is a multi-variable graph showing the relationship between concentrations of a high-magnetic-permeability material (450) with various cores as compared to a magnetic sensor that does not have a high-magnetic-permeability material (450). As shown in FIG. 6, each panel represents magnetic sensors (127) with varying concentrations of a high-magnetic-permeability material (450) core affixed. The first panel (602), titled "Large Mu", represents three test data samples for a magnetic sensor assembly with a large concentration of high-magnetic-permeability material (450) affixed. The first panel (602) can be representative of data collected with the position sensor (127) shown in FIGs. 5A and 5B having a comparatively large amount of high-magnetic-permeability material (450). The second panel (604), titled "No Mu", represents three test data samples for magnetic sensor assembly with no concentration of high-magnetic-permeability material (450) affixed. The third panel (606), titled "Small Mu", represents three test data samples for a magnetic sensor assembly having a small concentration of high-magnetic-permeability material (450) affixed, which can be understood as a concentration of high-magnetic-permeability material (450) that is greater than the second panel (604) and less than the first panel (602). The third panel (606) can be representative of data collected with the position sensor (127) shown in FIGs. 5C and 5D having a comparatively small amount of high-magnetic-permeability material (450).

As shown in FIG. 6, the magnetic sensitivity of the position sensor (127) having a large amount of high-magnetic-permeability material (450) had an increased sensitivity compared to the position sensor (127) having no or a small amount of high-magnetic-permeability material (450). The magnetic sensitivity is represented as "delta per mil" in the Y-axis with "delta per mil" being understood to mean change of magnetic sensitivity per millimeter of high-magnetic-permeability material (450). In other words, as the size of the high-magnetic-permeability material (450) increases, so does the sensitivity of the position sensor (127).

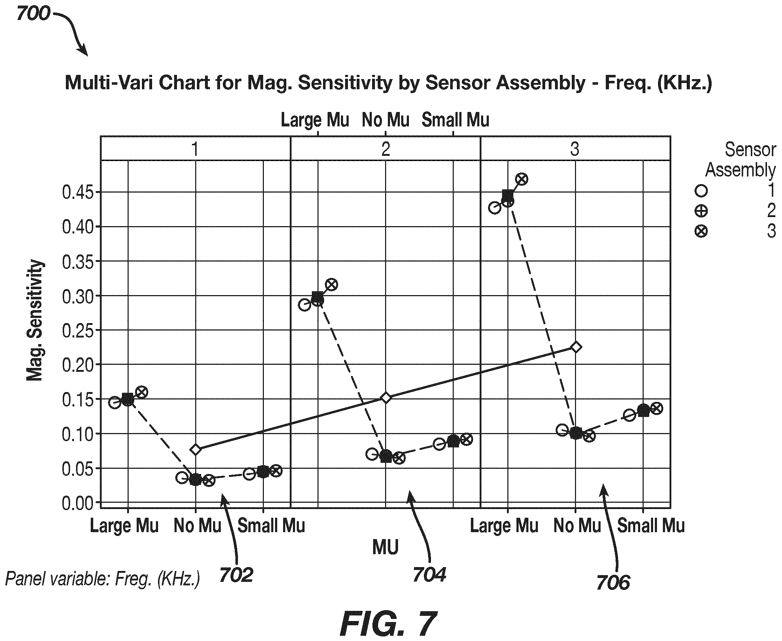

FIG. 7 is a multi-variable graph showing the relationship between magnetic sensitivity, concentration of high-magnetic-permeability materials (450), and frequency of the magnetic field. As shown in FIG. 7, each panel of the multi-variable graph examines the magnetic sensitivity of each magnetic sensor assembly based on a concentration of high-magnetic-permeability materials (450) at predetermined frequencies. The first panel (702) represents three data sets obtained with three different position sensor (127) assemblies at a first frequency, wherein each respective assembly has a high, no, or low concentration of high-magnetic-permeability materials (450). The second panel (704) represents three position sensor (127) assemblies at a second frequency, wherein each assembly has a high, no, or low concentration of high-magnetic-permeability materials (450). The third panel (706) represents three position sensor (127) assemblies at a third frequency, wherein each assembly has a high, no, or low concentration of high-magnetic-permeability materials (450). As shown in FIG. 7, the "Large Mu" concentrations at each of the frequencies resulted in the highest magnetic sensitivity when compared to the "Nu Mu" and "Small Mu" concentrations of high-magnetic-permeability materials (450). Furthermore, as shown in FIG. 7, the position sensor (127) assembly having a small concentration of high-magnetic-permeability materials (450) had a greater sensitivity than the position sensor (127) assembly that did not have any high-magnetic-permeability materials (450).

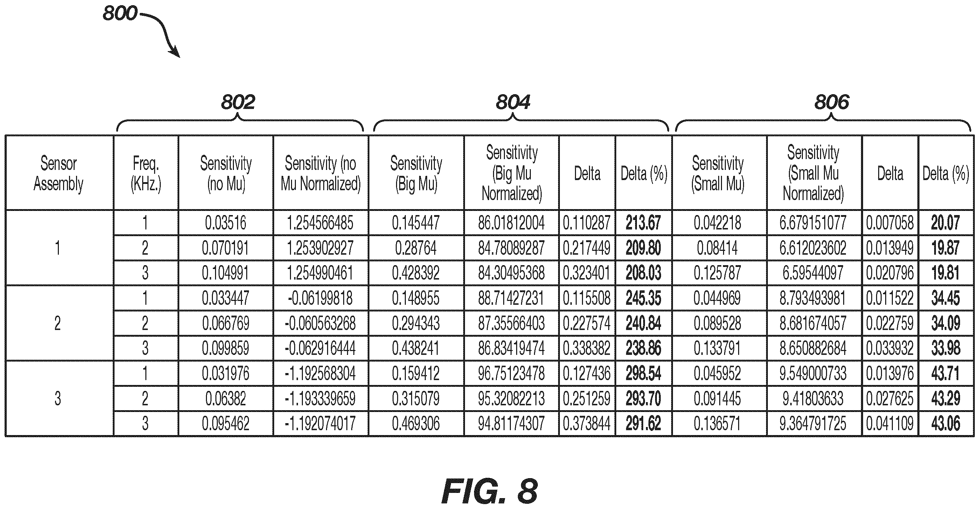

FIG. 8 is a table showing magnetic sensitivity of various concentrations of high-magnetic-permeability materials (450) for three sample position sensor (127) assemblies. Similar to FIG. 7, each panel of FIG. 8 provides tabular data representative of position sensor (127) assemblies having varying concentrations of high-magnetic-permeability materials (450) at predetermined frequencies. The first section (802) shows the magnetic sensitivity of a magnetic senor (127) with no concentration of high-magnetic-permeability materials (450), which can also be understood as "No Mu". The second section (804) shows the magnetic sensitivity of a magnetic senor (127) with a large concentration of high-magnetic-permeability materials (450), which can also be understood as "Big Mu". The third section (806) shows the magnetic sensitivity of a magnetic senor (127) with a small concentration of high-magnetic-permeability materials (450), which can also be understood as "Small Mu". Similar to the conclusions that can be drawn from FIG. 7, the magnetic sensitivities for magnetic sensors (127) with a "Big Mu" are shown to be higher than those for the "Small Mu" and "No Mu" position sensor (127) assemblies. In other words, increasing the size of the high-magnetic-permeability materials (450) affixed to the position sensor (127) can be understood as directly proportional to the overall magnetic sensitivity of the position sensor (127).

As shown in FIG. 8, the second section (804), which is representative of position sensor (127) assemblies similar to those shown in FIGs. 5A and 5B with large concentrations of high-magnetic-permeability materials (450), had greater magnetic sensitivity compared to the position sensor (127) having no or a small amount of high-magnetic-permeability material

(450). For example, each of the rows within the "Delta (%)" column of second section (804) of FIG. 8 shows values of over 200%, which is representative of significant increases in magnetic sensitivity for position sensor (127) assemblies with large concentrations of high-magnetic-permeability materials (450).

The third section (806) of FIG. 8, which is representative of position sensor (127) assemblies similar to those shown in FIG. 5C and 5D, had a higher magnetic sensitivity that position sensor (127) assemblies of the first section (802) having no high-magnetic-permeability material (450). However, the position sensor (127) assemblies of the third section (806) of FIG. 8 had a comparatively lesser magnetic sensitivity than position sensor (127) assemblies of the second section (804). For example, each row in the "Delta (%)" column of the third section (806) of FIG. 8 was less than each row in the "Delta (%)" column of the second section (804) but shows an increase over position sensors (127) not having high-magnetic-permeability material. In other words, any presence of high magnetic permeability materials (450) affixed on a position sensor (127) assembly increases the magnetic sensitivity of said position sensor (127) assembly.

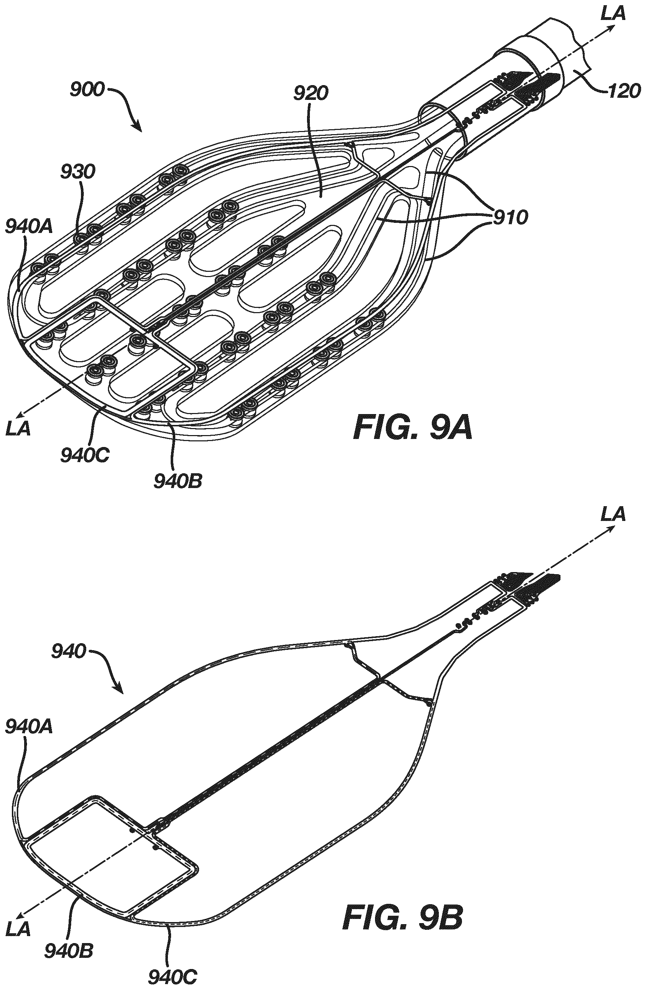

FIG. 9A is a perspective view of an exemplary end effector assembly (900) including electrodes (930) and a position sensor (127) assembly. FIG. 9B is a perspective view of the position sensor (127) assembly shown in FIG. 9A. The end effector assembly (900) may include a generally planar shape when deployed from a delivery sheath into an open configuration, as shown in FIG. 9A. It should be appreciated that the end effector assembly (900) can be in a closed configuration while compressed within the a delivery sheath and then transition to the deployed configuration shown in FIG. 9A. The end effector assembly (900) can be configured to transition to and maintain the geometry observed while in the open configuration, by having a support structure (910) formed from multiple spines. In some embodiments the support structure can be formed from Nitinol, which is known within the pertinent art to have shape memory characteristics. One method to shape set Nitinol can be through a time and temperature heat setting process to ensure the support structure (910) is configured to maintain a predetermined shape. In other words, the Nitinol support structure (910) as shown in FIG. 9A, can be shape set through a time and temperature heat setting process. It should be appreciated, however, that another suitable resilient material such as Polyetherimide (PEI) and the like may also be the material composition of the support structure (910).

The end effector assembly (900) may also include a polymer (920), which can be disposed across the support structure (910) and can assist in supporting the end effector assembly (900). The end effector assembly (900) may also include a plurality of electrodes (930) that can be disposed along the generally planar shape of the end effector assembly (900) in an arrangement of one or more rows, as shown in FIG. 9A. At least some of the plurality of electrodes (930) may be configured to detect electrophysiological signals when the end effector assembly (900) contacts tissue within the patient's (23) body. Alternatively, or in addition, at least some of the plurality of electrodes (930) can be configured to deliver ablative energy to tissue.

The end effector assembly (900) may also include a coil (940) that can be configured to generate an induced voltage when a magnetic field is applied to the end effector assembly (900). The coil (940) may also include a first coil (940A), a second coil (940B), and a third coil (940C). In some embodiments, the first coil (940A) can be disposed on a first side of the end effector assembly (900), the second coil (940B) can be disposed on a second side of the end effector assembly (900), and the third coil (940C) can be disposed near a distal end of the end effector assembly (900) as shown in FIG. 9B.

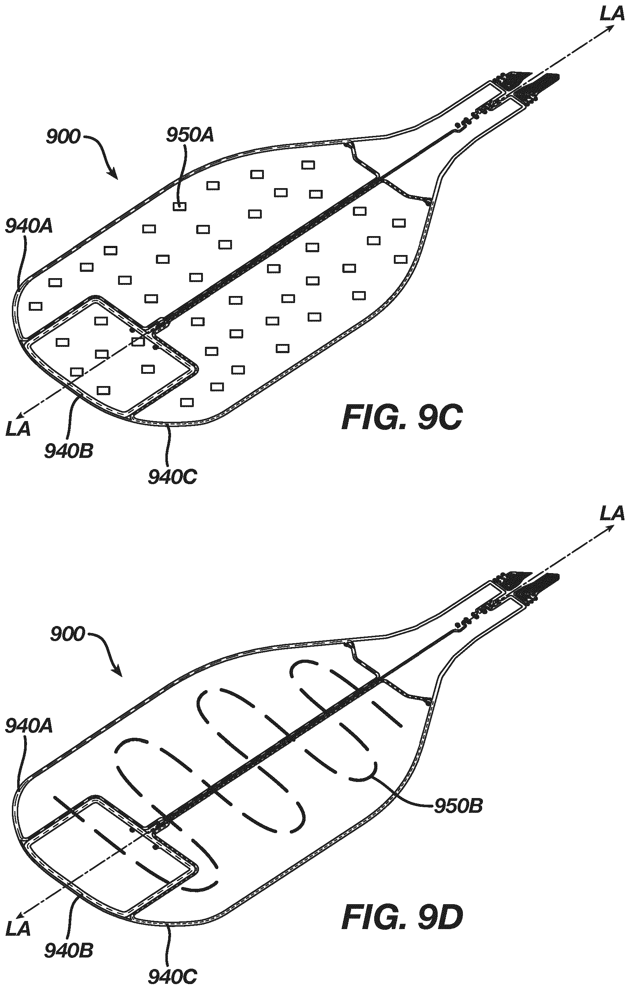

FIG. 9C and FIG. 9D are perspective views of the position sensor (127) of FIGs. 9A and 9B and high-magnetic-permeability materials. In some examples, a high-magnetic-permeability material (950A) can be impregnated into at least a portion of the end effector assembly (900) as shown in FIG. 9C. The impregnated high-magnetic permeability material (950A) can be disposed as discrete pieces of high-magnetic permeability material (950A) near the first coil (940A), the second coil (940B), and the third coil (940C) also as shown in FIG. 9C. In some examples, the end effector (900) can include high-magnetic-permeability material (950B) that is printed in a zig zag pattern onto the end effector assembly (900). The zig zag pattern may begin at a proximate end of the end effector assembly (900) and may span to a distal end of the end effector assembly (900) as shown in FIG. 9D.

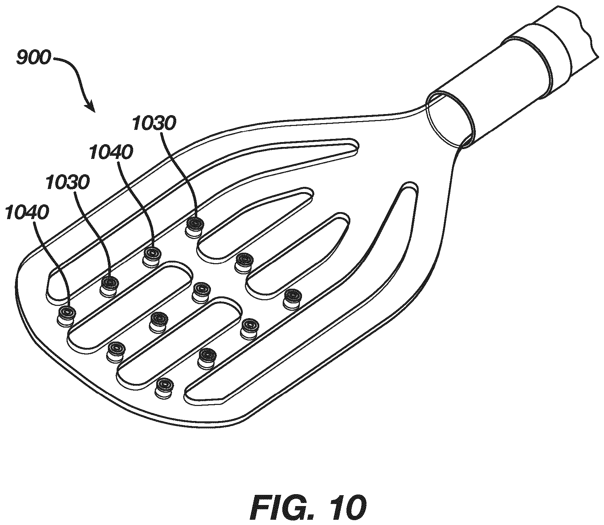

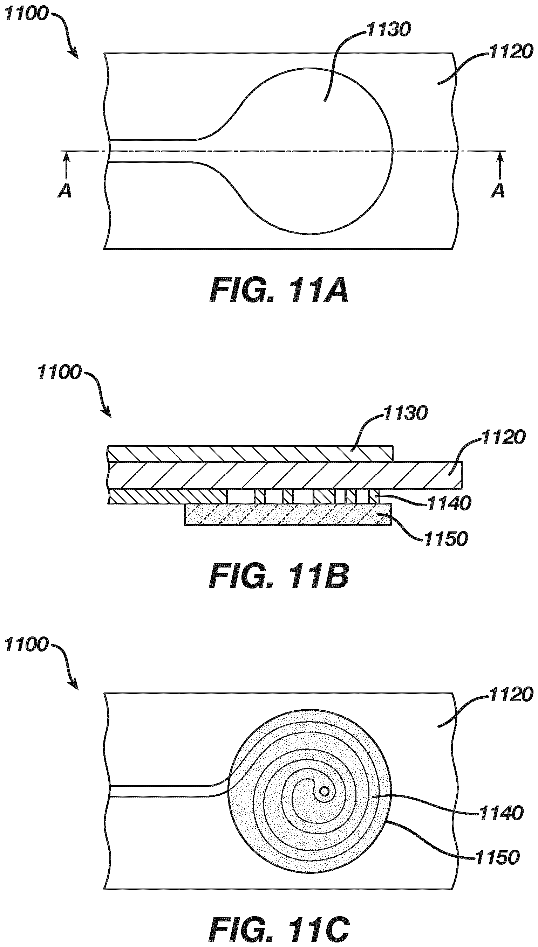

FIG. 10 is a perspective view of another exemplary end effector assembly (1000) with high-magnetic-permeability materials having sensor assemblies that are distributed in an alternating configuration. As shown in FIG. 10, an end effector assembly (1000) can have a body with a generally planar shape. In some embodiments, the end effector assembly (1000) can also have a plurality of first electrode assemblies (1030) and a plurality of second electrode assemblies (1040). The plurality of first electrode assemblies (1030) may be disposed along the body of the end effector (1000) and can be configured to detect electrophysiological signals and/or distribute ablative energy to tissue. The second plurality of electrode assemblies (1040) can also be disposed along the body of the end effector assembly (1000) and each of the second plurality of electrode assemblies (1040) can include an electrode (1130), a coil (1140) (as shown in FIG. 11), and a member (950) formed from a high-magnetic-permeability material (450).

Each electrode (1130) of the second plurality of electrode assemblies (1040) can be configured to detect electrophysiological signals and/or deliver ablative energy to tissue, as mentioned previously with the plurality of first electrode assemblies (1030). The coil (940) for each of the second plurality of electrode assemblies (1040) can be configured to generate an induced voltage when subjected to a magnetic field and may be disposed on the body of the end effector assembly (900). The member (950) formed from the high-magnetic-permeability material (450) for each of the second plurality of electrode assemblies (1040) can be disposed on at least one side of the body of the end effector assembly (900). In some embodiments, the first plurality of electrode assemblies (1030) and second plurality of electrode assemblies (1040) can be arranged in a horizontal alternating row pattern as shown in FIG. 10. It should be appreciated that the first plurality of electrode assemblies (1030) and second plurality of electrode assemblies (1040) can also be arranged in an alternating arrangement per row. In other words, each row can include one of a first plurality of electrode assemblies (1030) followed by one of a second plurality of electrode assemblies (1040) across a length (A) as shown in FIG. 10. It should also be appreciated, that the first plurality of electrode assemblies (1030) and second plurality of electrode assemblies (1040) can be arranged in a vertical alternating row pattern.

FIG. 11A is a top view of an electrode assembly (1100) having a high magnetic permeability material (1150). FIG. 11B is a cross-sectional view taken along line A-A of the electrode assembly (1100) shown in FIG. 11A having a high magnetic permeability material (1150). FIG. 11C is a bottom view of an electrode assembly having a high magnetic permeability material (1150) shown in FIG. 11A and 11B. In some embodiments, the electrode assembly (1100) may include an electrode (1130) that can be configured to detect electrophysiological signals. The electrode assembly (1130) may also include a planar substrate (1120) with the electrode (1130) disposed on a first side of the planar substrate (1120), as shown in FIG. 11A. The electrode assembly (1100) can also include an electromagnetic coil (1140) that can be disposed on a second side of the planar substrate (1120) as shown in FIG. 11B and FIG. 11C. The electromagnetic coil (1140) can be configured to provide a voltage when subjected to a magnetic field and may have a spiral shape that can have a width approximately equal to a width of the electrode (1130), as shown in FIG. 11C.

The electrode assembly (1100) can also include a high-magnetic-permeability member (1150). In some embodiments, the high-magnetic-permeability member (1150) can be disposed on a side of the electromagnetic coil (1140) opposite the planar substrate (1120), as shown in FIG. 11B. Alternatively, the high-magnetic-permeability member (1150) can be disposed between the electromagnetic coil (1140) and the planar substrate (1120). It should also be appreciated that the size of the high-magnetic-permeability member (1150) affixed to the electromagnetic coils (1140) can be directly proportional to the resultant magnetic sensitivity of the electromagnetic coils (1140). In other words, a larger high-magnetic-permeability member (1150) can result in increased magnetic sensitivity for the electromagnetic coils (1140), which can increase the position sensing performance of the electrode assembly (1100).

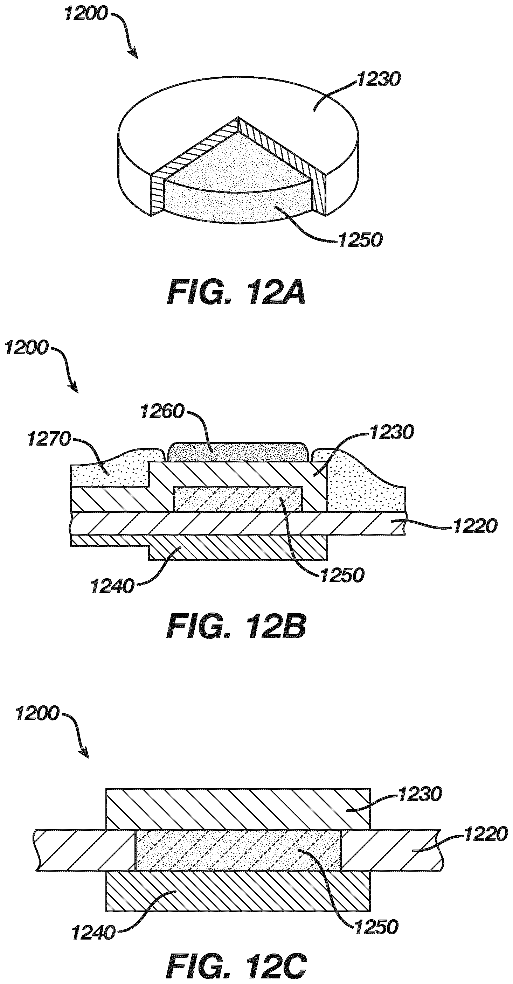

FIG. 12A is a section view of an electrode assembly (1200) having a high magnetic permeability material (1250)., FIG. 12B is a cross sectional view of an electrode assembly (1200) having a high magnetic permeability material (1250). FIG. 12C is an alternate section view of an electrode assembly (1200) having a high magnetic permeability material (1250).. In some examples, the electrode assembly (1200) can include an electrode (1230), a high-magnetic-permeability material member (1250), and an electromagnetic coil (1240) as shown in FIGs. 12A-12C. The electrode (1230), for example, may be gold-plated and the high-magnetic-permeability material member (1250) can be disposed at least partially within the electrode (1230), as shown in FIG. 12A. The electrode assembly (1200) can also include a conductive polymer (1260) that can be disposed on top of the electrode (1230) as shown in FIG. 12B. In some embodiments, the conductive polymer (1260) may be formed from the polymer poly(3,4-ethylenedioxythiophene) polystyrene sulfonate (PEDOT:PSS). It should be appreciated that there are other suitable conductive polymers known within the pertinent art that can be utilized with the present technology.

The electrode assembly (1200) can also include a cover lay biocompatible adhesive (1270) that may be disposed on top of the electrode (1230) and proximate the conductive polymer (1260) as shown in FIG. 12B. The electrode assembly (1200) may also include a substrate (1220) having a generally planar shape that may be disposed beneath the electrode (1230), as shown in FIG. 12B and FIG. 12C. In some embodiments, the high-magnetic-permeability material member (1250) can be disposed within the substrate (1220) between the electrode (1230) and the plurality of electromagnetic coils (1240) as shown in FIG. 12C. As mentioned previously, the plurality of electromagnetic coils (1240) can have a generally spiral shape that can have a width approximately equal to a width of the electrode (1230).

The disclosed technology described herein can be further understood according to the following clauses:
Clause 1: A magnetic location sensor comprising: first and second spiral coils disposed on a planar substrate, the first and second spiral coils disposed about respective first and second center axes of the first and second spiral coils and each of the first and second spiral coils configured to generate a voltage when subject to a magnetic field; and a high-magnetic-permeability planar member disposed near one of the first and second spiral coils so that when the planar coil is wrapped around a cylindrical substrate, the high-magnetic permeability planar member is disposed on an intersection of the first and second center axes.
Clause 2: The magnetic location sensor according to Clause 1, wherein the high-magnetic-permeability planar member comprises an area that is less than or equal to an area of the one of the first and second spiral coils.
Clause 3: The magnetic location sensor according to Clause 1, wherein the high-magnetic-permeability planar member comprises an area that is greater than or equal to an area of the one of the first and second spiral coils.
Clause 4: The magnetic location sensor according to any of Clauses 1-3, wherein the high-magnetic-permeability planar member comprises a Mu-metal.
Clause 5: The magnetic location sensor according to any of Clauses 1-3, wherein the high-magnetic-permeability planar member comprises a Metglas material.
Clause 6: The magnetic location sensor according to any of the preceding Clauses, wherein the high-magnetic-permeability planar member comprises a high-magnetic-permeability material that is printed along at least a portion of the planar substrate near the one of the first and second spiral coils.
Clause 7: The magnetic location sensor of Clause 6, wherein the high-magnetic-permeability material is printed in a zig-zag pattern along the planar substrate.
Clause 8: The magnetic location sensor of any of the preceding Clauses, wherein the magnetic location sensor further comprises a plurality of discrete high-magnetic-permeability members suspended in a non-conductive material along at least a portion of one of the first and second spiral coils.
Clause 9: An electrode assembly comprising: a planar substrate extending along a plane; an electrode disposed on the planar substrate and configured to detect electrophysiological signals; an electromagnetic coil disposed on the planar substrate and configured to generate a voltage when subject to a magnetic field; and a member comprising a high-magnetic-permeability material disposed near the electromagnetic coil.
Clause 10: The electrode assembly of Clause 9, wherein the electrode is disposed on a first side of the planar substrate and the electromagnetic coil is disposed on a second side of the planar substrate
Clause 11: The electrode assembly of Clause 10, wherein the member is disposed on a side of the electromagnetic coil opposite the planar substrate.
Clause 12: The electrode assembly of Clause 10, wherein the member is disposed between the electromagnetic coil and the planar substrate.
Clause 13: The electrode assembly of claim 10, wherein the member is disposed between the electrode and the planar substrate.
Clause 14: The electrode assembly of any of Clauses 9-13, wherein the electromagnetic coil comprises a spiral shape having a width approximately equal to a width of the electrode.
Clause 15: The electrode assembly of Clause 10, wherein the member is disposed at least partially in the substrate between the electrode and the electromagnetic coil.
Clause 16: The electrode assembly of Clause 10, wherein the member is disposed at least partially in the electrode on the first side of the substrate.
Clause 17: The electrode assembly of any of Clauses 9-16, further comprising a conductive polymer material disposed along an outer surface of the electrode.
Clause 18: The electrode assembly according to any of Clauses 9-17, wherein the high-magnetic-permeability material comprises a Mu-metal.
Clause 19: The electrode assembly according to any of Clauses 9-17, wherein the high-magnetic-permeability material comprises a Metglas material.
Clause 20: An end effector comprising: a plurality of electrodes disposed along the end effector and configured to detect electrophysiological signals; a coil disposed along the end effector and configured to generate a voltage when subject to a magnetic field; and
   a member disposed near the coil and comprising a high-magnetic-permeability material.
Clause 21: The end effector of Clause 21, the end effector comprising a generally planar shape, the plurality of electrodes disposed along the generally planar shape in one or more rows, and the coil disposed generally along an outer periphery of the generally planar shape.
Clause 22: The end effector of Clause 21, wherein the coil further comprises a first coil disposed on a first side of the end effector, a second coil disposed on a second side of the end effector, and a third coil disposed near a distal portion of the end effector.
Clause 23: The end effector of Clause 22, wherein the member comprises a high-magnetic-permeability material printed along at least a portion of the generally planar shape near at least one of the first coil, the second coil, and the third coil.
Clause 24: The end effector of Clause 23, wherein the high-magnetic-permeability material is printed in a zig-zag pattern from about a proximate end of the generally planar shape to about a distal end of the generally planar shape.
Clause 25: The end effector of any of Clauses 20-24, wherein the member further comprises a plurality of discrete members suspended in a non-conductive material along at least a portion of the generally planar shape.
Clause 26: The end effector of Clause 20 , wherein the end effector further comprises a substrate, wherein the plurality of electrodes are disposed on a first side of the substrate, and wherein the coil is disposed on a second side of the substrate near at least one electrode of the plurality of electrodes.
Clause 27: The end effector of Clause 26, wherein the member is disposed on a side of the coil opposite the substrate.
Clause 28: The end effector of Clause 26, wherein the member is disposed between the coil and the substrate.
Clause 29: The end effector of Clause 26, wherein the member is disposed between the electrode and the substrate.
Clause 30: The end effector of any of Clauses 26-29, wherein the coil comprises a generally spiral shape having a width approximately equal to a width of the electrode.
Clause 31: The end effector of claim 26, wherein the member is disposed at least partially in the substrate between the electrode and the coil.
Clause 32: The end effector of claim 26, wherein the member is disposed at least partially in the electrode on the first side of the substrate.
Clause 33: The end effector of any of Clauses 26-32, further comprising a conductive polymer material disposed along an outer surface of the electrode.
Clause 34: The end effector according to any of Clauses 20-33, wherein the high-magnetic-permeability material comprises a Mu-metal.
Clause 35: The end effector according to any of Clauses 20-33, wherein the high-magnetic-permeability material comprises a Metglas material.
Clause 36: The end effector according to any of Clauses 20-35 further comprising an insulative substrate disposed between the plurality of electrodes and the coil.
Clause 37: The end effector according to any of Clauses 20-36 further comprising an insulative material disposed between the plurality of electrodes and the member.
Clause 38: A end effector comprising: a body having a generally planar shape; a plurality of first electrode assemblies disposed along the body and configured to detect electrophysiological signals; a plurality of second electrode assemblies disposed along the body, each electrode assembly of the plurality of second electrode assemblies comprising: an electrode configured to detect electrophysiological signals; a coil disposed on the body and configured to generate a voltage when subject to a magnetic field; and a member comprising a high-magnetic-permeability material disposed on at least one side of the body.
Clause 39: The end effector of Clause 38, wherein the plurality of first electrode assemblies and the plurality of second electrode assemblies are disposed in an alternating pattern along the body.
Clause 40: The end effector of Clauses 38 or 39, wherein the plurality of first electrode assemblies and the plurality of second electrode assemblies are disposed on a first side of the body and on a second side of the body in an alternating pattern.
Clause 41: The end effector of any of Clauses 38-40, wherein the alternating pattern comprises a plurality of first electrode assemblies arranged in a first row and a plurality of second electrode assemblies arranged in a second row.
Clause 42: The end effector of any of Clauses 38-40, wherein each electrode assembly of the plurality of second electrode assemblies comprises: the electrode disposed on a first side of a substrate; and the coil disposed on a second side of the substrate.
Clause 43: The end effector of Clause 42, wherein the member is disposed on a side of the coil opposite the substrate.
Clause 44: The end effector of Clause 42, wherein the member is disposed between the coil and the substrate.
Clause 45: The end effector of Clause 42, wherein the member is disposed between the electrode and the substrate.
Clause 46: The end effector of any of Clauses 42-45, wherein the coil comprises a spiral shape having a width approximately equal to a width of the electrode.
Clause 47: The end effector of Clause 42, wherein the member is disposed at least partially in the substrate between the electrode and the coil.
Clause 48: The end effector of Clause 42, wherein the member is disposed at least partially in the electrode on the first side of the substrate.
Clause 49: The end effector of any of Clauses 42-48, further comprising a conductive polymer material disposed along an outer surface of the electrode.
Clause 50: The end effector according to any of Clauses 38-49, wherein the high-magnetic-permeability material comprises a Mu-metal.
Clause 51: A end effector comprising: a plurality of flexible spines, the plurality of flexible spines configured to transition the end effector between an expanded configuration and a collapsed configuration; a plurality of electrode assemblies disposed along the plurality of flexible spines, each electrode assembly of the plurality of electrode assemblies comprising: a substrate extending at least partially along a spine of the plurality of flexible spines; an electrode disposed on a first side of the substrate and configured to detect electrophysiological signals; a coil disposed on a second side of the substrate and configured to generate a voltage when subject to a magnetic field; and a member comprising a high-magnetic-permeability material.
Clause 52: The end effector of Clause 51, wherein the member is disposed on a side of the coil opposite the substrate.
Clause 53: The end effector of Clause 51, wherein the member is disposed between the coil and the substrate.
Clause 54: The end effector of Clause 51, wherein the member is disposed between the electrode and the substrate.
Clause 55: The end effector of any of Clauses 51-54, wherein the coil comprises generally spiral shape having a width approximately equal to a width of the electrode.
Clause 56: The end effector of Clause 51, wherein the member is disposed at least partially in the substrate between the electrode and the coil.
Clause 57: The end effector of Clause 51, wherein the member is disposed at least partially in the electrode on the first side of the substrate.
Clause 58: The end effector of any of Clauses 51-57, further comprising a conductive polymer material disposed along an outer surface of the electrode.
Clause 59: The end effector according to any of Clauses 51-58, wherein the high-magnetic-permeability material comprises a Mu-metal.
Clause 60: The end effector according to any of Clauses 51-58, wherein the high-magnetic-permeability material comprises a Metglas material.

Any of the examples or embodiments described herein may include various other features in addition to or in lieu of those described above. The teachings, expressions, embodiments, examples, etc. described herein should not be viewed in isolation relative to each other. Various suitable ways in which the teachings herein may be combined should be clear to those skilled in the art in view of the teachings herein.

Having shown and described exemplary embodiments of the subject matter contained herein, further adaptations of the methods and systems described herein may be accomplished by appropriate modifications without departing from the scope of the claims. In addition, where methods and steps described above indicate certain events occurring in certain order, it is intended that certain steps do not have to be performed in the order described but in any order as long as the steps allow the embodiments to function for their intended purposes. Therefore, to the extent there are variations of the invention, which are within the spirit of the disclosure or equivalent to the inventions found in the claims, it is the intent that this patent will cover those variations as well. Some such modifications should be apparent to those skilled in the art. For instance, the examples, embodiments, geometrics, materials, dimensions, ratios, steps, and the like discussed above are illustrative. Accordingly, the claims should not be limited to the specific details of structure and operation set forth in the written description and drawings.

## Claims

1. A magnetic location sensor comprising:
first and second spiral coils disposed on a planar substrate, the first and second spiral coils disposed about respective first and second center axes of the first and second spiral coils and each of the first and second spiral coils configured to generate a voltage when subject to a magnetic field; and
a high-magnetic-permeability planar member disposed near one of the first and second spiral coils so that when the planar coil is wrapped around a cylindrical substrate, the high-magnetic-permeability planar member is disposed on an intersection of the first and second center axes.

2. The magnetic location sensor according to claim 1, wherein the high-magnetic-permeability planar member comprises an area that is i) less than or equal to an area of the one of the first and second spiral coils, or ii) greater than or equal to an area of the one of the first and second spiral coils.

3. The magnetic location sensor of claim 1 or claim 2, wherein the high-magnetic-permeability planar member comprises a Mu-metal, or a Metglas material.

4. The magnetic location sensor of any preceding claim, wherein the high-magnetic-permeability planar member comprises a high-magnetic-permeability material that is printed along at least a portion of the planar substrate near the one of the first and second spiral coils, optionally wherein the high-magnetic-permeability material is printed in a zig-zag pattern along the planar substrate.

5. The magnetic location sensor of any preceding claim, wherein the magnetic location sensor further comprises a plurality of discrete high-magnetic-permeability members suspended in a non-conductive material along at least a portion of one of the first and second spiral coils.

6. An electrode assembly comprising:
a planar substrate extending along a plane;
an electrode disposed on the planar substrate and configured to detect electrophysiological signals;
an electromagnetic coil disposed on the planar substrate and configured to generate a voltage when subject to a magnetic field; and
a member comprising a high-magnetic-permeability material disposed near the electromagnetic coil.

7. The electrode assembly of claim 6, wherein the electrode is disposed on a first side of the planar substrate and the electromagnetic coil is disposed on a second side of the planar substrate.

8. The electrode assembly of claim 7, wherein the member is disposed i) on a side of the electromagnetic coil opposite the planar substrate, ii) between the electromagnetic coil and the planar substrate, or iii) between the electrode and the planar substrate.

9. The electrode assembly of any of claims 6 to 8, wherein the electromagnetic coil comprises a spiral shape having a width approximately equal to a width of the electrode.

10. An end effector comprising:
a plurality of electrodes disposed along the end effector and configured to detect electrophysiological signals;
a coil disposed along the end effector and configured to generate a voltage when subject to a magnetic field; and
a member disposed near the coil and comprising a high-magnetic-permeability material.

11. The end effector of claim 10, the end effector comprising a generally planar shape, the plurality of electrodes disposed along the generally planar shape in one or more rows, and the coil disposed generally along an outer periphery of the generally planar shape.

12. The end effector of claim 11, wherein the coil further comprises a first coil disposed on a first side of the end effector, a second coil disposed on a second side of the end effector, and a third coil disposed near a distal portion of the end effector.

13. The end effector of claim 12, wherein the member comprises a high-magnetic-permeability material printed along at least a portion of the generally planar shape near at least one of the first coil, the second coil, and the third coil.

14. The end effector of claim 13, wherein the high-magnetic-permeability material is printed in a zig-zag pattern from about a proximate end of the generally planar shape to about a distal end of the generally planar shape.

15. The end effector of any of claims 10 to 14, wherein the member further comprises a plurality of discrete members suspended in a non-conductive material along at least a portion of the generally planar shape.
